# EUROPÄISCHE PATENTSCHRIFT

(11) **EP 2 411 564 B1**
(45) Veröffentlichungstag und Bekanntmachung des Hinweises auf die Patenterteilung: **10.05.2017**
(21) Anmeldenummer: 10709552.3
(22) Anmeldetag: 22.03.2010
(51) Int. Cl.: C25B 3/02, C07C 43/174, C07C 43/303, C07C 45/51

(54) **ELEKTROCHEMISCHES VERFAHERN ZUR HERSTELLUNG VON 3-TERT.-BUTYLBENZALDEHYD-DIMETHYLACETAL**
ELECTROCHEMICAL PROCESS FOR PRODUCING 3-TERT-BUTYLBENZALDEHYDE-DIMETHYLACETAL
PROCÉDÉ ÉLECTROCHIMIQUE DE PRODUCTION DE 3-TERT-BUTYLBENZALDEHYDE-DIMETYLACETALS

(30) Priorität: 27.03.2009 EP 09156398
(43) Veröffentlichungstag der Anmeldung: 01.02.2012
(73) Patentinhaber: BASF SE, 67056 Ludwigshafen am Rhein (DE)
(72) Erfinder: STECKER, Florian, 68167 Mannheim (DE); FISCHER, Andreas, 64646 Heppenheim (DE); BOTZEM, Jörg, 67117 Limburgerhof (DE); GRIESBACH, Ulrich, 68305 Mannheim (DE); PELZER, Ralf, 37699 Fürstenberg (DE)
(74) Vertreter: BASF IP Association
(86) Internationale Anmeldenummer: PCT/EP2010/053656
(87) Internationale Veröffentlichungsnummer: WO 2010/108874

(56) Entgegenhaltungen:
- EP-A2- 0 287 954
- DATABASE PUBCHEM [Online] 5. Dezember 2007 (2007-12-05), "Compound Summmary" XP002588536 gefunden im ncbi Database accession no. CID 20782896

## Beschreibung

Die Erfindung betrifft 3-tert.-Butylbenzaldehyddimethylacetal sowie ein elektrochemisches Verfahren zur Herstellung von 3-tert.-Butyl-benzaldehyddimethylacetal.

Es ist bekannt, substituierte Benzaldehyddimethylacetale direkt durch elektrochemische Methoxylierung der entsprechenden Toluole herzustellen. P. Loyson, S. Gouws, B. Barton, M. Ackermann, S. Afr. J. Chem 2004, 57, 53-56 beschreiben ein derartiges Verfahren. Nachteilig an der elektrochemischen Seitenkettenmethoxylierung von Toluolen ist, dass nur mit elektronenschiebenden Resten wie tert-Butyl, Methyl oder Alkoxy substituierte Toluole in wirtschaftlich interessanten Ausbeuten methoxyliert werden können. Reste wie Ethyl, Isopropyl oder Isobutyl wirken zwar ebenfalls elektronenschiebend, ihre benzylischen Protonen können aber ebenfalls bei der elektrochemischen Umsetzung in einer Nebenreaktion durch Methoxygruppen substituiert werden. So lässt sich p-Cymol nicht glatt zum Cuminaldehyd-Dimethylacetal methoxylieren, wie in F. Vaudano, P. Tissot, Electrochimica Acta 2001, 46, 875-880 beschrieben, da auch stets die Isopropylgruppe anteilig methoxyliert wird. Substituierte Benzaldehyddimethylacetale und die diesen zugrunde liegenden Aldehyde sind wichtige Zwischenprodukte z.B. in der Synthese von 2-Methyl-3-Phenylpropanal-Riechstoffen wie z.B. Cyclamenaldehyd, Lysmeral^{®}(BASF SE) oder Silvial.

Die EP 0 129 795 A2 beschreibt ein Verfahren zur Herstellung von substituierten Benzaldehyddialkylacetalen durch Elektrooxidation von entsprechend substituierten Alkyltoluolen bei der man einen Elektrolyten verwendet, der 50 bis 90 Gew.% an einem entsprechenden Alkanol, 8.5 bis 40 Gew.% an dem Alkyltoluol und 0,01 bis 1,5 Gew.% an einer HO₃S-Gruppen enthaltenden Säure enthält.

Die EP 0 554 564 A1 offenbart ein Verfahren zur Herstellung von substituerten Benzaldehydacetalen, wobei die Substituenten des Aromaten mindestens ein benzylisches Wasserstoffatom aufweisen, durch elektrochemische Oxidation eines entsprechenden Benzylethers in Gegenwart eines entsprechenden Alkanols sowie in Gegenwart eines Hilfselektrolyten, indem man im sauren, neutralen oder schwach basischen Bereich elektrolysiert.

Die EP 0 638 665 A1 offenbart ein Verfahren zur Herstellung von substituierten Benzaldehyddialkylacetalen durch elektrochemische Oxidation entsprechend substituierter Toluolverbindungen indem man eine substituierte Toluolverbindung in Gegenwart eines Alkanols und eines Hilfselektrolyten in einer Elektrolysezelle oxidiert, die so erhaltene Reaktionslösung außerhalb der Elektrolysezelle auf einen Druck entspannt, der 10 mbar bis 10 bar geringer ist als der Druck in der Elektrolysezelle.

Die EP 0 287 954 A2 offenbart die Herstellung von 3-tert-Butyl-4-methoxy-benzaldehydedimethylacetal durch elektrochemische anodische Methoxylierung von 3-tert-Butyl-4-methoxy-toluol in Gegenwart von Methanol.

Aufgabe der vorliegenden Erfindung war es, ein elektrochemisches Verfahren zur Herstellung von 3-tert.-Benzaldehyddimethylacetal bereitzustellen, das sich durch eine sehr gute Produktausbeute, durch eine sehr gute Produktselektivität und durch eine hohe Stromausbeute auszeichnet und dabei unter Einsatz wohlfeiler Ausgangsstoffe und Reagenzien bzw. Hilfsstoffe gut im technischen Maßstab durchführbar ist.

Gelöst wurde die Aufgabe durch die Bereitstellung eines Verfahrens zur Herstellung von 3-tert.-Butylbenzaldehyddimethylacetal der Formel (I) 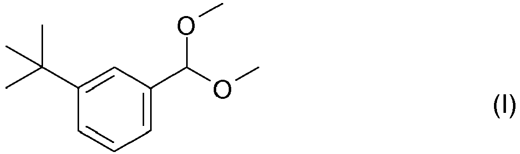 durch elektrochemische anodische Methoxylierung von 3-tert.-Butyltoluol der Formel (II) 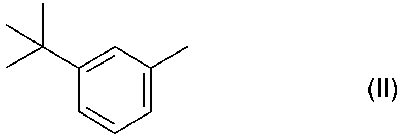 und/oder 3-tert.-Butyl-methylbenzylverbindungen der Formel (III), 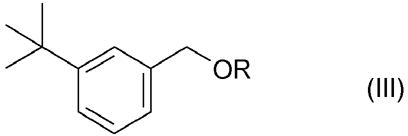 in denen der Rest
- R: Methyl oder C(O)R' bedeutet, wobei R' für einen geradkettigen oder verzweigten Alkylrest mit 1 bis 6 Kohlenstoffatomen steht,
und/oder Di-(3-tert.-butylbenzyl)ether der Formel (IV) 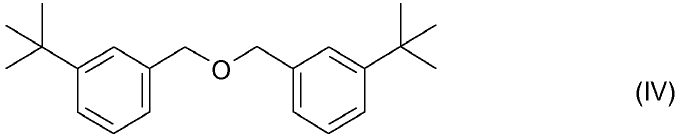 in einer Elektrolyselösung umfassend Methanol, mindestens ein Leitsalz sowie gegebenenfalls ein Cosolvens oder mehrere verschiedene Cosolventien.

Als Ausgangsstoff zur Durchführung des erfindungsgemäßen Verfahrens dienen wahlweise die Verbindungen 3-tert.-Butyltoluol der Formel (II) 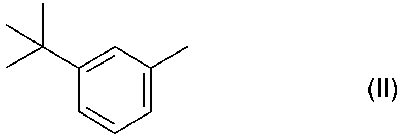 und/oder die 3-tert.-Butyl-methylbenzylverbindungen der Formel (III), d.h. 3-tert.-Butyl-methylbenzylether und/oder die Ester der Formel (III), 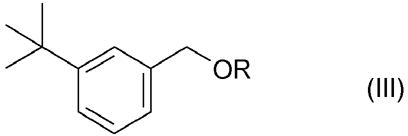 in denen der Rest
- R: Methyl oder C(O)R' bedeutet, wobei R' für einen geradkettigen oder verzweigten Alkylrest mit 1 bis 6 Kohlenstoffatomen steht,
und/oder Di-(3-tert.-butylbenzyl)ether der Formel (IV)

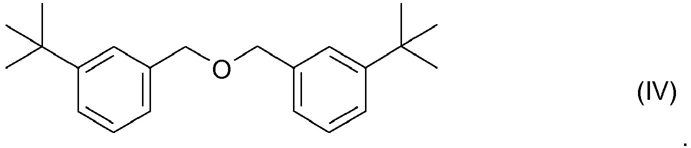

Die genannten Verbindungen können im Rahmen des erfindungsgemäßen Verfahrens im Prinzip jeweils alleine oder auch in Form jedweder Gemische von zwei oder allen drei der genannten Verbindungen eingesetzt werden.

Unter den erfindungsgemäß einsetzbaren Verbindungen der Formel (III) ist insbesondere der 3-tert.-Butyl-methylbenzylether der Formel (IIIa) 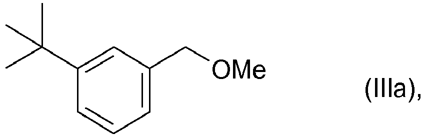 worin Me Methyl bedeutet, bevorzugt. Im Fall der ebenfalls erfindungsgemäß einsetzbaren Ester der Formel (III) kann der Rest R' für einen geradkettigen oder verzweigten Alkylrest mit 1 bis 6 Kohlenstoffatomen, bevorzugt für Methyl, Ethyl, Propyl oder Isopropyl, insbesondere bevorzugt für Methyl stehen. Eine erfindungsgemäß ebenfalls bevorzugte Ausgangsverbindung ist dementsprechend das Acetat der Formel (IIIb)

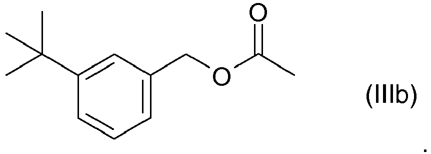

Im Rahmen einer bevorzugten Ausführungsform des erfindungsgemäßen Verfahrens setzt man lediglich 3-tert.-Butyltoluol der Formel (II) als Ausgangsstoff ein. Im Verlauf der erfindungsgemäßen elektrochemischen anodischen Methoxylierung können sich daraus zunächst die genannten Verbindungen der Formeln (III), insbesondere 3-tert.-Butyl-methylbenzylether der Formel (IIIa) 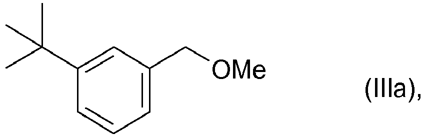 worin Me Methyl bedeutet, bilden, die dann ihrerseits unter den Reaktionsbedingungen zum gewünschten Produkt 3-tert.-Butylbenzaldehyddimethylacetal der Formel (I) abreagieren bzw. weiter umgesetzt werden.

Das als Ausgangsstoff im Rahmen des erfindungsgemäßen Verfahrens bevorzugte 3-tert.-Butyltoluol der Formel (II) kann nach bekannten Verfahren, beispielsweise durch Isomerisierung des para-substituierten Isomers hergestellt werden, wie beispielsweise in der JP 2738093 beschrieben.

Der 3-tert.-Butylbenzylmethylether der Formel (IIIa) kann beispielsweise durch Methoxymethylierung von tert.-Butylbenzol mit Formaldehyd-Dimethylacetal an einem Zeolith-Katalysator hergestellt werden.

Die Herstellung substituierter Benzylmethylether allgemein aus Alkylbenzolen (Toluol, Ethylbenzol, Isobutylbenzol, Cumol, tert.-Butylbenzol etc.) durch Umsetzung mit Formaldehyd-Dimethylacetal an einem Zeolith-Katalysator ist in DE 199 04 900 A1 beschrieben. Bei niedrigen Umsätzen (< 30%) werden gute Produktselektivitäten erhalten, bei höheren Umsätzen überwiegt die Reaktion zum Diarylmethan. Weitere dem Fachmann bekannte Möglichkeiten zur Herstellung des Benzylmethylethers der Formel (II) sind die Methylierung von 3-tert.-Butylbenzylalkoholen oder die Umsetzung von 3-tert.-Butylbenzylhalogeniden mit Methanol bzw. Methanolaten in einer Williamson-Ethersynthese.

In einer weiteren Ausführungsform des erfindungsgemäßen Verfahrens setzt man den Di-(3-tert.-butylbenzyl)ether der Formel (IV) 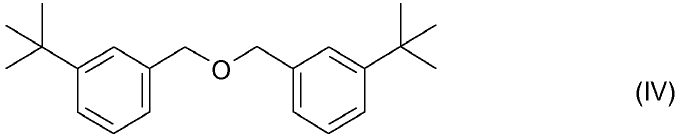 ein, entweder allein oder wie vorstehehend beschrieben in Form eines Gemisches mit den weiteren möglichen Ausgangsstoffen der Formeln (II) und/oder (III). Dabei wird aus einem Äquivalent des Dibenzylethers (IV) zwei Äquivalente 3-tert.-Butylbenzaldehyddimethylacetal der Formel (I) gebildet.

Der Dibenzylether der Formel (IV) kann beispielsweise durch Umsetzung des entsprechend substituierten Benzylalkohols der Formel (V) 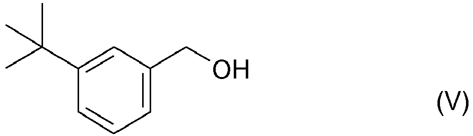 mit dem entsprechend substituierten Benzylhalogenid der allgemeinen Formel (VI) 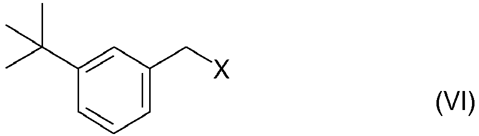 hergestellt werden. Dabei wird meist zur Deprotonierung der Alkohole in Gegenwart von Basen gearbeitet (Fileti, Gazz. Chim. Ital. 1884, 14, 498-501). Die Ethersynthese kann auch durch sauer katalysierte Kondensation zweier Moleküle Benzylalkohol ausgeführt werden (Fileti, Gazz. Chim. Ital. 1882, 12, 501; F. Shirini, M.A. Zolfigol, K. Mohammadi, Phosphorus, Sulfur Silicon Relat. Elem. 2003, 178 (11), 2357-2362). Für diese Reaktion gibt es zahlreiche weitere Beispiele in der Literatur.

Der Di-(3-tert.-butylbenzyl)ether der Formel (IV) kann auch durch Funktionalisierung von unsubstituiertem Dibenzylether nach einschlägigen Verfahren, die dem Fachmann bekannt sind, wie elektrophile aromatische Substitution oder Friedel-Crafts-Alkylierung, hergestellt werden. Dibenzylether ist ein großtechnisches Produkt, das unter anderem als Weichmacher eingesetzt wird.

Das Auftreten von Alkylbenzylmethylethern als Zwischenstufen der elektrochemischen Methoxylierung von Alkyltoluolen, so als Zwischenstufen der Methoxylierung von p-tert-Butyltoluol bzw. p-Xylol, ist in P. Loyson, S. Gouws, B. Zeelie, S. Afr. J. Chem., 2002, 55, 125-131 bzw. P. Loyson, S. Gouws, B. Barton, M. Ackermann, S. Afr. J. Chem., 2004, 57, 53-56 beschrieben. Der Eintritt der ersten Methoxygruppe ist dabei der geschwindigkeitsbestimmende Schritt, der infolge dessen mit nur mäßiger Ausbeute verläuft.

Bei der elektrochemischen Methoxylierung des Dibenzylethers der Formel (IV) wird direkt das Benzaldehyd-Dimethylacetale der Formel (I) erhalten, wobei die Dibenzylether-Bismethoxylierungs-Zwischenstufe (VII) durchlaufen wird. Diese ist jedoch unter den Reaktionsbedingungen nicht beständig und setzt sich mit Methanol unter Freisetzung von Wasser zur Verbindung der Formel (I) um.

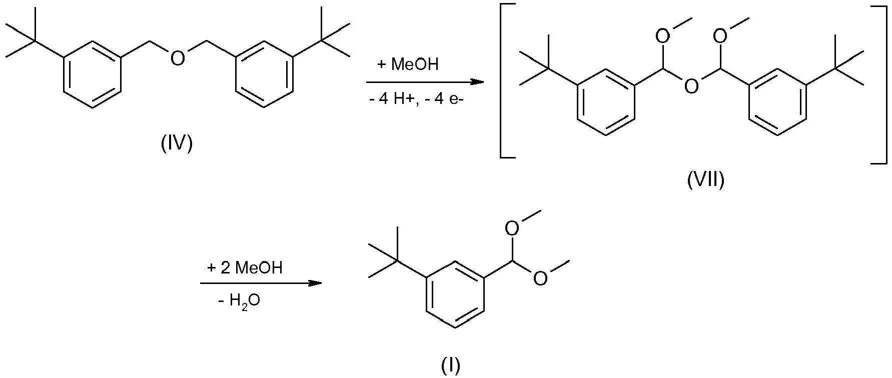

Die Elektrolyselösung enthält im Rahmen des erfindungsgemäßen Verfahrens neben den Gewählten Ausgangsstoffe der Formeln (II), (III) und/oder (IV) mindestens Methanol sowie mindestens ein Leitsalz.

Bei den Leitsalzen, die in der Elektrolyselösung enthalten sein können, handelt es sich im Allgemeinen um Alkali-, Tetra(C₁- bis C₆-alkyl)ammonium-, bevorzugt Tri(C₁- bis C₆-alkyl)methylammoniumsalze. Als Gegenionen kommen Sulfat, Hydrogensulfat, Alkylsulfate, Arylsulfate, Alkylsulfonate, Arylsulfonate, Halogenide, Phosphate, Carbonate, Alkylphosphate, Alkylcarbonate, Nitrat, Alkoholate, Tetrafluorborat oder Perchlorat in Betracht.

Weiterhin kommen die von den vorstehend genannten Anionen abgeleiteten Säuren als Leitsalze in Betracht, also zum Beispiel Schwefelsäure, Sulfonsäuren sowie Carbonsäuren.

Daneben eignen sich als Leitsalze auch ionische Flüssigkeiten. Geeignete ionische Flüssigkeiten sind beschrieben in "Ionic Liquids in Synthesis", Hrsg. Peter Wasserscheid, Tom Welton, Verlag Wiley VCH, 2003, Kap. 1 bis 3 sowie in der DE-A 102004011427.

Bevorzugte Leitsalze sind im Rahmen des erfindungsgemäßen Verfahrens Methyltributylammoniummethylsulfat, Methyltriethylammoniummethylsulfat, Natriummethylsulfat, Natriummethansulfonat und Schwefelsäure, insbesondere bevorzugt Natriummethansulfonat, Methyltributylammoniummethylsulfat und Methyltriethylammoniummethylsulfat, noch mehr bevorzugt Methyltributylammoniummethylsulfat und Methyltriethylammoniummethylsulfat und ganz besonders bevorzugt Methyltributylammoniummethylsulfat. Die genannten Leitsalze, insbesondere Methyltributylammoniummethylsulfat und Methyltriethylammoniummethylsulfat können alleine oder in Form von Gemischen untereinander eingesetzt werden.

Im Rahmen einer bevorzugten Ausführungsform des erfindungsgemäßen Verfahrens setzt man als Leitsalz Methyltributylammoniummethylsulfat und/oder Methyltriethylammoniummethylsulfat ein. Insbesondere bevorzugt setzt man als Leitsalz Methyltributylammoniummethylsulfat ein. Wiederum bevorzugt setzt man die genannten Leitsalze alleine oder in Form eines Gemisches zweier verschiedener Leitsalze, bevorzugt jedoch alleine ein.

Im Rahmen einer vorteilhaften Ausführungsform des erfindungsgemäßen Verfahrens wählt man die Konzentration des Leitsalzes in der Elektrolyselösung im Bereich von 0,1 bis 20 Gewichtsprozent (Gew.-%), bevorzugt im Bereich von 0,2 bis 15 Gew.-%, noch mehr bevorzugt von 0,25 bis 10 Gew.-%, noch mehr bevorzugt von 0,5 bis 7,5 Gew.-% und insbesondere bevorzugt im Bereich von 1 bis 5 Gew.-%.

Eine weitere bevorzugte Ausführungsform des erfindungsgemäßen Verfahrens ist dadurch gekennzeichnet, dass man die elektrochemische anodische Methoxylierung bei einer Temperatur der Elektrolyselösung im Bereich von 35 bis 70°C, bevorzugt im Bereich von 45 bis 60°C durchführt.

Darüber hinaus führt man das erfindungsgemäße Verfahren bevorzugt so durch, dass man die elektrochemische anodische Methoxylierung bei einem Absolutdruck im Bereich von 500 bis 100000 mbar, bevorzugt bei einem Absolutdruck im Bereich von 1000 bis 4000 mbar durchführt.

Gegebenenfalls setzt man der Elektrolyselösung übliche Cosolventien zu. Dabei handelt es sich um die in der organischen Chemie allgemein üblichen inerten Lösungsmittel mit einem hohen Oxidationspotential. Beispielhaft genannt seien Dimethylcarbonat oder Propylencarbonat. Im Rahmen einer bevorzugten Ausführungsform führt man das erfindungsgemäße Verfahren daher in Gegenwart von Dimethylcarbonat und/oder Propylencarbonat als Cosolventien durch.

Als Cosolvens ist grundsätzlich auch Wasser geeignet, der Anteil von Wasser im Elektrolyten beträgt bevorzugt weniger als 20 Gew.-%.

Das erfindungsgemäße Verfahren kann in allen üblichen geteilten oder ungeteilten Elektrolysezellentypen durchgeführt werden. Es kann mit gutem Erfolg sowohl diskontinuierlich als auch kontinuierlich durchgeführt werden. Im Rahmen einer bevorzugten Ausführungsform führt man das erfindungsgemäße Verfahren kontinuierlich durch. Vorzugsweise arbeitet man kontinuierlich mit ungeteilten Durchflusszellen.

Ganz besonders geeignet sind bipolar geschaltete Kapillarspaltzellen oder Plattenstapelzellen, bei denen die Elektroden als Platten ausgestaltet sind und planparallel angeordnet sind (Ullmann's Encyclopedia of Industrial Chemistry, 1999 electronic release, Sixth Edition, VCH-Verlag Weinheim, Volume Electrochemistry, Chapter 3.5 special cell designs sowie Chapter 5, Organic Electrochemistry, Subchapter 5.4.3.2 Cell Design). Als Elektrodenmaterial sind Edelmetalle wie Platin, Mischoxidelektroden wie RuOxTiOx (sogenannte DSA-Elektroden) oder kohlenstoffhaltige Materialien wie Graphit, Glassy Carbon oder Diamantelektroden bevorzugt. Ganz besonders bevorzugt werden Graphitelektroden eingesetzt. Im Rahmen einer bevorzugten Ausführungsform führt man das erfindungsgemäße Verfahren unter Verwendung einer Plattenstapelzelle durch.

Die Stromdichten, bei denen man das Verfahren durchführt, betragen im Allgemeinen 1 bis 1000 mA/cm², bevorzugt 10 bis 100 mA/cm². Besonders bevorzugt wird das Verfahren bei Stromdichten zwischen 10 und 50 mA/cm² durchgeführt. Im Allgemeinen wird bei Normaldruck gearbeitet. Höhere Drücke werden bevorzugt dann angewandt, wenn bei höheren Temperaturen gearbeitet werden soll, um ein Sieden der Ausgangsverbindungen bzw. des Lösungsmittels zu vermeiden.

Als Anodenmaterialien eignen sich beispielsweise Edelmetalle wie Platin oder Metalloxide wie Ruthenium oder Chromoxid oder Mischoxide des Typs RuOₓ, TiOₓ sowie Diamantelektroden. Bevorzugt sind Graphit oder Kohleelektroden.

Als Kathodenmaterialien kommen beispielsweise Eisen, Stahl, Edelstahl, Nickel oder Edelmetalle wie Platin sowie Graphit oder Kohlematerialien sowie Diamantelektroden in Betracht. Bevorzugt ist das System Graphit als Anode und Kathode sowie Graphit als Anode und Nickel, Edelstahl oder Stahl als Kathode.

Nach Beendigung der Reaktion wird die Elektrolytlösung nach allgemeinen Trennmethoden aufgearbeitet. Hierzu wird die Elektrolyselösung im Allgemeinen zunächst destilliert und die einzelnen Verbindungen werden in Form von unterschiedlichen Fraktionen getrennt gewonnen. Eine weitere Reinigung kann beispielsweise durch Kristallisation, Extraktion, Destillation oder chromatographisch erfolgen.

Ein weiterer Aspekt der vorliegenden Erfindung betrifft ein Verfahren zur Herstellung des 3-tert.-Butylbenzaldehyd der Formel (VIII) 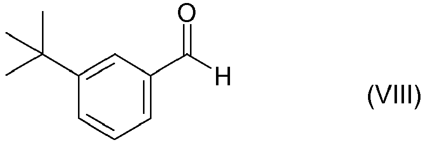 umfassend die Herstellung von 3-tert.-Butylbenzaldehyddimethylacetal der Formel (I) gemäß dem vorstehend beschriebenen Verfahren und anschließender Hydrolyse. Die genannte Hydrolyse kann nach dem Fachmann an sich bekannten Verfahren, beispielsweise durch einfaches Inkontaktbringen der Verbindung der Formel (I) mit Wasser oder einer Säure wie beispielsweise verdünnter Salz-, Schwefel- oder auch Essigsäure bewerkstelligt werden.

Die vorliegende Erfindung betrifft in einem weiteren Aspekt 3-tert.-Butylbenzaldehyddimethylacetal der Formel (I)

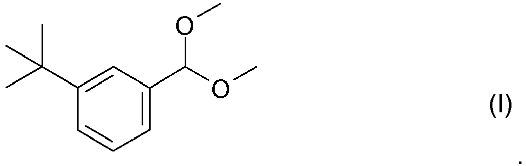

Die vorliegende Erfindung betrifft in einem weiteren Aspekt 3-tert.-Butyl-methylbenzylether der Formel (IIIa) 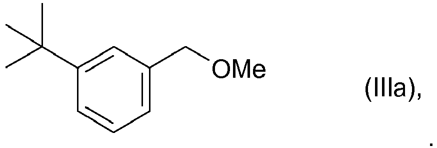 worin Me Methyl bedeutet.

Die vorliegende Erfindung betrifft in einem weiteren Aspekt Di-(3-tert.-butylbenzyl)ether der Formel (IV)

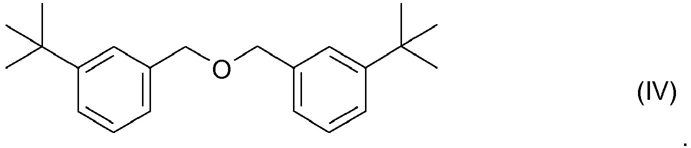

Die vorliegende Erfindung betrifft in einem weiteren Aspekt schließlich die als Intermediat durchlaufene Verbindung der Formel (VII) 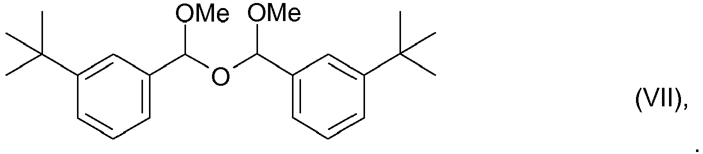 worin Me Methyl bedeutet.

Die Erfindung wird durch die nachstehenden Beispiele näher erläutert.

### Beispiele 1 bis 5: Herstellung von 3-tert.-Butylbenzaldehyddimethylacetal der Formel (I)

### Beispiel 1:

Zur elektrochemischen Methoxylierung von 3-tert-Butyltoluol der Formel (II) wurde ein Elektrolyt bestehend aus 105 g der Verbindung der Formel (II), 1,9 g Schwefelsäure (96-98 %) als Leitsalz und 593,1 g Methanol bei 45°C und einer Stromdichte von 34 mA/cm² so lange in einer ungeteilten Kapillarspaltzelle mit 10 runden Grafitelektroden (A = 32 cm²) elektrolysiert, bis eine Ladungsmenge von 15 F/mol der Verbindung der Formel (II) erreicht war. Die destillative Aufarbeitung ergab 33,3 g der Verbindung der Formel (I) sowie 7,5 g 3-tert-Butylbenzylmethylether, entsprechend einer Gesamtausbeute von Acetal und Ether von 30%.

### Beispiel 2:

Zur elektrochemischen Methoxylierung der Verbindung der Formel (II) wurde ein Elektrolyt bestehend aus 140 g der Verbindung der Formel (II), 1,9 g Schwefelsäure (96-98%) und 3,47 g Natriummethylat (30% in Methanol) als Leitsalz und 554,6 g Methanol bei 45°C und einer Stromdichte von 22 mA/cm² so lange in einer ungeteilten Kapillarspaltzelle mit 10 runden Grafitelektroden (A = 32 cm²) elektrolysiert, bis eine Ladungsmenge von 15 F/mol der Verbindung der Formel (II) erreicht war. Die destillative Aufarbeitung ergab 54,5 g der Verbindung der Formel (I) sowie 17,0 g 3-tert-Butylbenzylmethylether, entsprechend einer Gesamtausbeute von Acetal und Ether von 38%.

### Beispiel 3:

Zur elektrochemischen Methoxylierung der Verbindung der Formel (II) wurde ein Elektrolyt bestehend aus 105 g der Verbindung der Formel (II), 14 g Methyltributylammoniummethylsulfat (MTBS, 60% in Methanol) als Leitsalz und 581 g Methanol bei 53°C und einer Stromdichte von 22 mA/cm² so lange in einer ungeteilten Kapillarspaltzelle mit 10 runden Grafitelektroden (A = 32 cm²) elektrolysiert, bis eine Ladungsmenge von 18 F/mol der Verbindung der Formel (II) erreicht war. Die destillative Aufarbeitung ergab 48,3 g der Verbindung der Formel (I) sowie 10,5 g 3-tert-Butylbenzylmethylether, entsprechend einer Gesamtausbeute von Acetal und Ether von 41 %.

### Beispiel 4:

Zur elektrochemischen Methoxylierung der Verbindung der Formel (II) wurde ein Elektrolyt bestehend aus 14 g der Verbindung der Formel (II), 1,4 g Methyltriethylammoniummethylsulfat (MTES) als Leitsalz und 54,6 g Methanol bei 53°C und einer Stromdichte von 22 mA/cm² so lange in einer ungeteilten Becherglastopfzelle (A = 10 cm²) elektrolysiert, bis eine Ladungsmenge von 14 F/mol der Verbindung der Formel (II) erreicht war. Die destillative Aufarbeitung ergab 9,99 g der Verbindung der Formel (I) sowie 0,54 g 3-tert-Butylbenzylmethylether, entsprechend einer Gesamtausbeute von Acetal und Ether von 54%.

### Beispiel 5:

Zur elektrochemischen Methoxylierung der Verbindung der Formel (II) wurde ein Elektrolyt bestehend aus 14 g der verbindung der Formel (II), 0,42 g Natriummethansulfonat als Leitsalz und 55,6 g Methanol bei 53°C und einer Stromdichte von 22 mA/cm² so lange in einer ungeteilten Becherglastopfzelle (A = 10 cm²) elektrolysiert, bis eine Ladungsmenge von 19 F/mol der Verbindung der Formel (II) erreicht war. Die destillative Aufarbeitung ergab 8,75 g der Verbindung der Formel (I) sowie 0,42 g 3-tert-Butylbenzylmethylether, entsprechend einer Gesamtausbeute von Acetal und Ether von 47%.

## Patentansprüche

1. Verfahren zur Herstellung von 3-tert.-Butylbenzaldehyddimethylacetal der Formel (I) 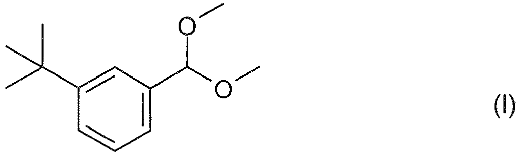 durch elektrochemische anodische Methoxylierung von 3-tert.-Butyltoluol der Formel (II) 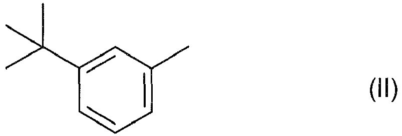 und/oder 3-tert.-Butylbenzylverbindungen der Formel (III), 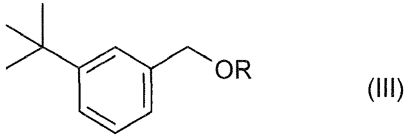 in denen der Rest
R Methyl oder C(O)R' bedeutet, wobei R' für einen geradkettigen oder verzweigten Alkylrest mit 1 bis 6 Kohlenstoffatomen steht,
und/oder Di-(3-tert.-butylbenzyl)ether der Formel (IV) 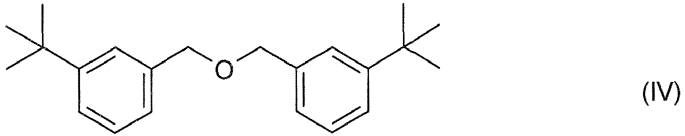 in einer Elektrolyselösung umfassend Methanol, mindestens ein Leitsalz sowie gegebenenfalls ein Cosolvens oder mehrere verschiedene Cosolventien.

2. Verfahren nach Anspruch 1, **dadurch gekennzeichnet, dass** man lediglich 3-tert.-Butyltoluol der Formel (II) als Ausgangsstoff einsetzt.

3. Verfahren nach Anspruch 1 oder 2, **dadurch gekennzeichnet, dass** man als Leitsalz Methyltributylammoniummethylsulfat und/oder Methyltriethylammoniummethylsulfat einsetzt.

4. Verfahren nach einem der Ansprüche 1 bis 3, **dadurch gekennzeichnet, dass** man als Leitsalz Methyltributylammoniummethylsulfat einsetzt.

5. Verfahren nach einem der Ansprüche 1 bis 4, **dadurch gekennzeichnet, dass** man als Colsovens Dimethylcarbonat und/oder Propylencarbonat einsetzt.

6. Verfahren nach einem der Ansprüche 1 bis 5, **dadurch gekennzeichnet, dass** man die Konzentration des Leitsalzes in der Elektrolyselösung im Bereich von 0,1 bis 20 Gewichtsprozent (Gew.-%) wählt.

7. Verfahren nach einem der Ansprüche 1 bis 6, **dadurch gekennzeichnet, dass** man die elektrochemische anodische Methoxylierung bei einer Temperatur der Elektrolyselösung im Bereich von 35 bis 70°C durchführt.

8. Verfahren nach einem der Ansprüche 1 bis 7, **dadurch gekennzeichnet, dass** man die elektrochemische anodische Methoxylierung bei einem Absolutdruck im Bereich von 500 bis 100000 mbar durchführt.

9. Verfahren nach einem der Ansprüche 1 bis 8, **dadurch gekennzeichnet, dass** man die elektrochemische anodische Methoxylierung bei einer Stromdichte im Bereich von 10 bis 100 mA/cm² durchführt.

10. Verfahren nach einem der Ansprüche 1 bis 9, **dadurch gekennzeichnet**, das man die elektrochemische anodische Methoxylierung unter Verwendung einer Plattenstapelzelle durchführt.

11. Verfahren nach einem der Ansprüche 1 bis 10, **dadurch gekennzeichnet, dass** man die elektrochemische anodische Methoxylierung kontinuierlich durchführt.

12. Verfahren zur Herstellung von 3-tert.-Butylbenzaldehyd der Formel (VIII) 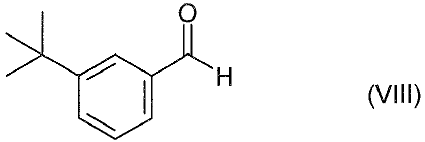 umfassend die Herstellung von 3-tert.-Butylbenzaldehyddimethylacetal der Formel (I) gemäß einem der Ansprüche 1 bis 11 und anschließender Hydrolyse.

13. 3-tert.-Butylbenzaldehyddimethylacetal.

14. Di-(3-tert.-butylbenzyl)ether der Formel (IV)

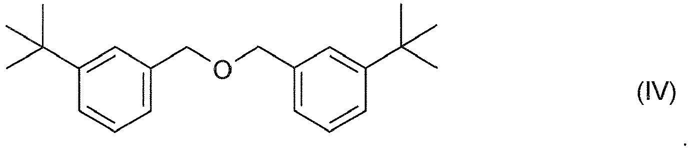

15. Verbindung der Formel (VII) 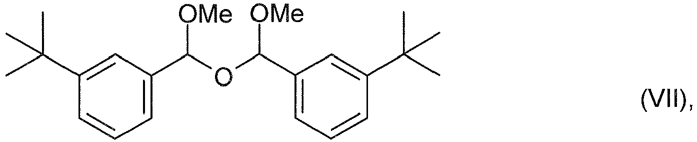 worin Me Methyl bedeutet.

## Claims

1. A method for producing 3-tert-butylbenzaldehyde dimethyl acetal of the formula (I) 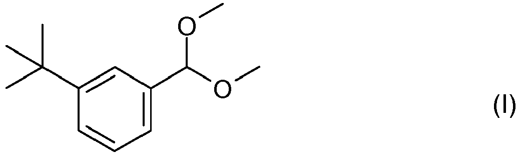 by electrochemical anodic methoxylation of 3-tert-butyltoluene of the formula (II) 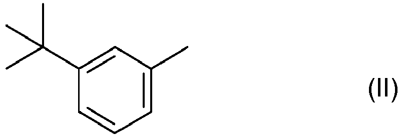 and/or 3-tert-butylbenzyl compounds of the formula (III), 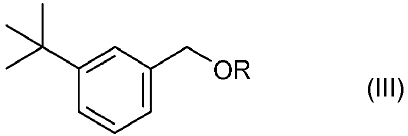 in which the radical
R is methyl or C(O)R', where R' is a straight-chain or branched alkyl radical having 1 to 6 carbon atoms,
and/or di(3-tert-butylbenzyl) ether of the formula (IV) 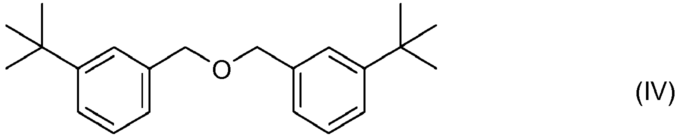 in an electrolysis solution comprising methanol, at least one conductive salt and optionally one cosolvent or two or more different cosolvents.

2. The method according to claim 1, wherein only 3-tert-butyltoluene of the formula (II) is used as starting material.

3. The method according to claim 1 or 2, wherein the conductive salt used is methyltributylammonium methyl sulfate and/or methyltriethylammonium methyl sulfate.

4. The method according to any one of claims 1 to 3, wherein the conductive salt used is methyltributylammonium methyl sulfate.

5. The method according to any one of claims 1 to 4, wherein the cosovent used is dimethyl carbonate and/or propylene carbonate.

6. The method according to any one of claims 1 to 5, wherein the concentration of the conductive salt in the electrolysis solution is chosen in the range from 0.1 to 20 percent by weight (% by wt).

7. The method according to any one of claims 1 to 6, wherein the electrochemical anodic methoxylation is carried out at a temperature of the electrolysis solution in the range from 35 to 70°C.

8. The method according to any one of claims 1 to 7, wherein the electrochemical anodic methoxylation is carried out at an absolute pressure in the range from 500 to 100 000 mbar.

9. The method according to any one of claims 1 to 8, wherein the electrochemical anodic methoxylation is carried out at a current density in the range from 10 to 100 mA/cm².

10. The method according to any one of claims 1 to 9, wherein the electrochemical anodic methoxylation is carried out using a stacked plate cell.

11. The method according to any one of claims 1 to 10, wherein the electrochemical anodic methoxylation is carried out continuously.

12. A method for producing 3-tert-butylbenzaldehyde of the formula (VIII) 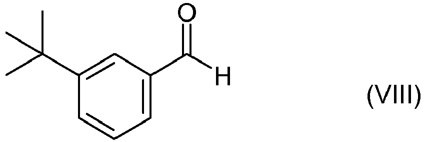 comprising the preparation of 3-tert-butylbenzaldehyde dimethyl acetal of the formula (I) according to any one of claims 1 to 11 and subsequent hydrolysis.

13. 3-tert-Butylbenzaldehyde dimethyl acetal.

14. Di(3-tert-butylbenzyl) ether of the formula (IV)

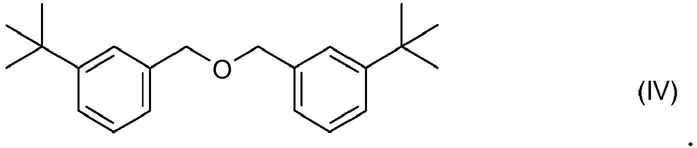

15. A compound of the formula (VII) 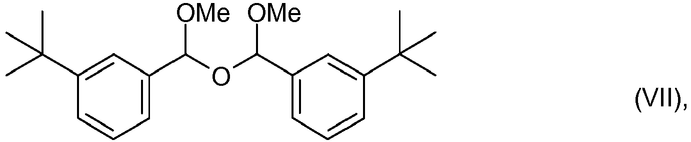 in which Me is methyl.

## Revendications

1. Procédé de fabrication de 3-tert.-butylbenzaldéhyde-diméthylacétal de formule (I) 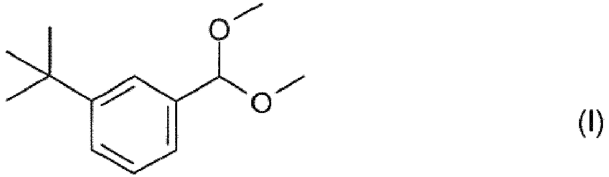 par méthoxylation anodique électrochimique de 3-tert.-butyltoluène de formule (II) 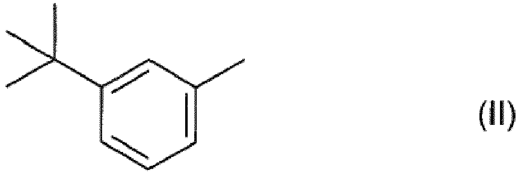 et/ou de composés de 3-tert.-butylbenzyle de formule (III) 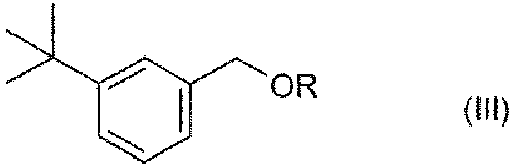 dans laquelle le radical
R signifie méthyle ou C(O)R', R' représentant un radical alkyle linéaire ou ramifié de 1 à 6 atomes de carbone,
et/ou d'éthers de di-(3-tert.-butylbenzyle) de formule (IV) 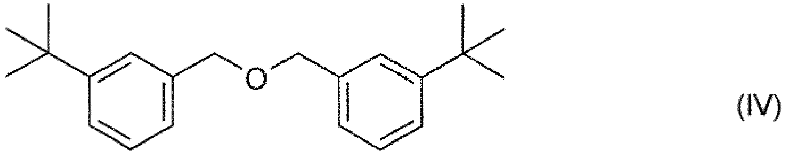
dans une solution d'électrolyse comprenant du méthanol, au moins un sel conducteur et éventuellement un co-solvant ou plusieurs co-solvants différents.

2. Procédé selon la revendication 1, **caractérisé en ce qu'**uniquement du 3-tert.-butyltoluène de formule (II) est utilisé en tant que matière première.

3. Procédé selon la revendication 1 ou 2, **caractérisé en ce que** du méthylsulfate de méthyltributylammonium et/ou du méthylsulfate de méthyltriéthylammonium sont utilisés en tant que sel conducteur.

4. Procédé selon l'une quelconque des revendications 1 à 3, **caractérisé en ce que** du méthylsulfate de méthyltributylammonium est utilisé en tant que sel conducteur.

5. Procédé selon l'une quelconque des revendications 1 à 4, **caractérisé en ce que** du carbonate de diméthyle et/ou du carbonate de propylène sont utilisés en tant que co-solvant.

6. Procédé selon l'une quelconque des revendications 1 à 5, **caractérisé en ce que** la concentration du sel conducteur dans la solution d'électrolyse est choisie dans la plage allant de 0,1 à 20 pour cent en poids (% en poids).

7. Procédé selon l'une quelconque des revendications 1 à 6, **caractérisé en ce que** la méthoxylation anodique électrochimique est réalisée à une température de la solution d'électrolyse dans la plage allant de 35 à 70°C.

8. Procédé selon l'une quelconque des revendications 1 à 7, **caractérisé en ce que** la méthoxylation anodique électrochimique est réalisée à une pression absolue dans la plage allant de 500 à 100 000 mbar.

9. Procédé selon l'une quelconque des revendications 1 à 8, **caractérisé en ce que** la méthoxylation anodique électrochimique est réalisée à une densité de courant dans la plage allant de 10 à 100 mA/cm².

10. Procédé selon l'une quelconque des revendications 1 à 9, **caractérisé en ce que** la méthoxylation anodique électrochimique est réalisée en utilisant une cellule à empilement de plaques.

11. Procédé selon l'une quelconque des revendications 1 à 10, **caractérisé en ce que** la méthoxylation anodique électrochimique est réalisée en continu.

12. Procédé de fabrication de 3-tert.-butylbenzaldéhyde de formule (VIII) 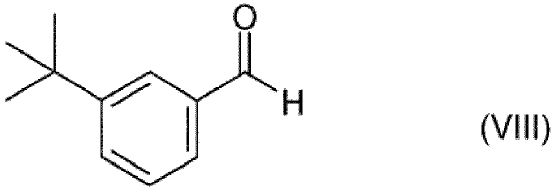 comprenant la fabrication de 3-tert.-butylbenzaldéhydediméthylacétal de formule (I) selon l'une quelconque des revendications 1 à 11, puis une hydrolyse.

13. 3-tert.-Butylbenzaldéhyde-diméthylacétal.

14. Éther de di-(3-tert.-butylbenzyle) de formule (IV)

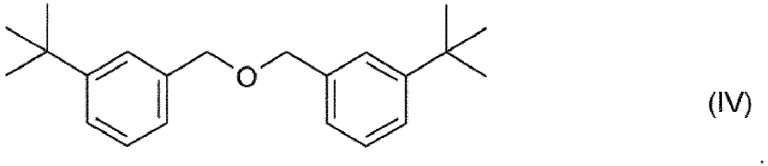

15. Composé de formule (VII) 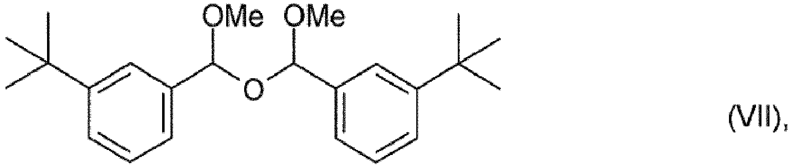 dans laquelle Me signifie méthyle.
